# EUROPEAN PATENT SPECIFICATION

(11) **EP 3 669 904 B1**
(45) Date of publication and mention of the grant of the patent: **17.06.2026**
(21) Application number: 19217779.8
(22) Date of filing: 19.12.2019
(51) Int. Cl.: A61M 1/02, A61M 1/36

(54) **METHODS AND SYSTEMS FOR PLATELET CRYOPRESERVATION**
VERFAHREN UND SYSTEME ZUR THROMBOZYTENKRYOKONSERVIERUNG
PROCÉDÉS ET SYSTÈMES POUR CRYOCONSERVATION DE PLAQUETTES

(30) Priority: 21.12.2018 US 201862784045 P
(43) Date of publication of application: 24.06.2020
(73) Proprietor: Fenwal, Inc., Lake Zurich, IL 60047 (US)
(72) Inventor: MIN, Kyungyoon, Lake Zurich, IL Illinois 60047 (US); KARPIEL, Adrienne, Lake Zurich, IL Illinois 60047 (US); RADWANSKI, Katherine, Lake Zurich, IL Illinois 60047 (US); HEBER, Cheryl, Lake Zurich, IL Illinois 60047 (US)
(74) Representative: Maikowski & Ninnemann Patentanwälte Partnerschaft mbB

(56) References cited:
- WO-A2-2008/035240
- RU-C1- 2 623 081
- US-A- 4 764 463

## Description

### Field of the Disclosure

The present disclosure is directed to methods and systems for preparing platelets for cryopreserved storage. More particularly, the present disclosure is directed to methods for preparing a platelet product for cryopreservation and kits for use in the preparation of a platelet cryopreservation product, and in the thawing of a cryopreserved platelet product.

### Background

Whole blood is made up of various cellular components such as red blood cells, white blood cells and platelets suspended in its liquid component, plasma. Whole blood can be separated into its constituent components (cellular or liquid), and the desired separated component can be administered to a patient in need of that particular component. For example, platelets can be removed from the whole blood of a healthy donor, collected, and later administered to a cancer patient, whose ability to "make" platelets has been compromised by chemotherapy or radiation treatment.

Commonly, platelets are collected by introducing whole blood into a centrifuge chamber wherein the whole blood is separated into its constituent components, including platelets, based on the size and densities of the different components. This requires that the whole blood be passed through a centrifuge after it is withdrawn from, and before it is returned to, the donor. Typical blood processing systems thus include a permanent, reusable centrifuge assembly containing the hardware (drive system, pumps, valve actuators, programmable controller) that spins and pumps the blood, and a disposable, sealed and sterile fluid processing assembly that is mounted cooperatively on the hardware. The centrifuge assembly spins a disposable centrifuge chamber in the fluid processing assembly during a collection procedure, thereby separating the blood into its constituent components.

"On line" automated blood separation systems are commonly used today to collect large numbers of platelets. On line systems perform the separation steps necessary to separate platelets from whole blood in a sequential process with the donor present. On line systems draw whole blood from the donor, separate out the desired platelets from the drawn blood, and return the remaining red blood cells and plasma to the donor, all in a sequential flow loop. Large volumes of whole blood can be processed using an automated on line system. Due to the large processing volumes, large yields of concentrated platelets can be collected. Moreover, since the donor's red blood cells are returned, the donor can donate platelets for on line processing much more frequently.

In the automated, on-line separation and collection of platelets, sometimes referred to as platelet apheresis or simply "plateletpheresis", the platelets are separated from whole blood and concentrated in the centrifuge chamber or elsewhere in the fluid processing set (hereinafter "platelet concentrate" or "PC"). Although most of the plasma is removed during apheresis, a small volume of plasma may still remain in the PC, hereinafter referred to as "residual plasma". The platelets are typically reconstituted in a liquid medium, such as plasma and/or a synthetic storage solution, for storage until needed for transfusion to a patient. Platelets may be collected by known automated apheresis devices, such as the Amicus^{®} Separator, available from Fenwal, Inc., of Lake Zurich, III, a subsidiary of Fresenius-Kabi of Bad Homburg, Germany.

Currently, platelets may be stored for five or even seven days at room temperature (e.g., 22°C). After seven days, however, platelet function may become impaired. For longer term storage platelets may be frozen or cryopreserved. In cryopreservation, platelets are typically combined with a cryopreservative solution that protects the platelets during freezing. Typically, in the preparation of cryopreserved platelets, a cryopreservative solution is combined with previously collected platelets by joining (in a sterile manner) the source of the cryopreservative solution to the container of platelets. Once mixed, the combined platelets and cryopreservative are typically subjected to centrifugation to reduce the volume. After centrifugation and volume reduction, the platelets and cryopreservative are transferred to a container suitable for freezing. After the designated storage period, the cryopreserved platelets are thawed and then combined with a platelet additive solution for storage prior to transfusion. RU 2623081C1 discloses a system of containers: one of frozen platelets and the other of plasma. Both containers are heated in a defroster and combined by sterile connection. WO 2008/035240 refers to a platelet freezing system including a closed circuit. US 4764463 deals with platelet cryopreservation.

While the above-described method of preparing cryopreserved platelets allows for extending the time between platelet collection and platelet transfusion, the method does require several manual steps and sterile docking steps. Thus, it would be desirable to provide a method of preparing platelets for cryopreservation and subsequent thawing that requires fewer manual connection steps.

### Summary

The invention is set out in the appended set of claims. Accordingly, the present disclosure is directed to a cryopreservation kit. The kit includes a container of a platelet additive solution, a platelet storage container and tubing interconnecting the platelet container with said platelet storage container and said additive solution for establishing a flow paths therebetween. The kit includes a docking site for joining a platelet collection container that includes a platelet concentrate. The kit further includes a storage unit having a housing including one or more compartments for holding and organizing the platelet collection chamber, the container of platelet additive solution and the platelet storage container. The storage unit is made of a material that can withstand the temperatures of cryopreservation, allowing the platelets to freeze, and also allows frozen platelets to thaw in a relatively short time.

### Brief Description of the Drawings

Figure 1 is a perspective view of an automated apheresis device that may be used in the collection and processing of platelets for cryopreservation in accordance with the present disclosure;
Figure 2 is an enlarged perspective view of the front panel of the device of FIG. 3 with an exemplary disposable fluid circuit for collecting platelets mounted on the device;
Figure 3 is a diagram of the disposable fluid circuit useful in collecting and processing platelets for cryopreservation in accordance with the present disclosure;
Figure 4 is a perspective view of processing chamber of the disposable fluid circuit of Fig. 3;
Figure 5 is a schematic diagram of a cryopreservation kit in accordance with present disclosure;
Figure 6 is a schematic diagram of a storage unit/cassette for housing the cryopreservation kit of Fig. 5;
Figure 7 is a schematic diagram of a storage unit/cassette of Fig. 6 with the cryopreservation kit and platelet concentration chamber attached thereto and housed in the unit/cassette;
Figure 8 is a schematic diagram of an alternative embodiment of a storage unit/cassette for housing the cryopreservation kit of Fig. 5; and
Figure 9 is a schematic diagram of the storage unit/cassette of Fig. 8 with the cryopreservation kit and platelet concentration chamber attached thereto housed in the cassette

### Detailed Description of the Embodiments

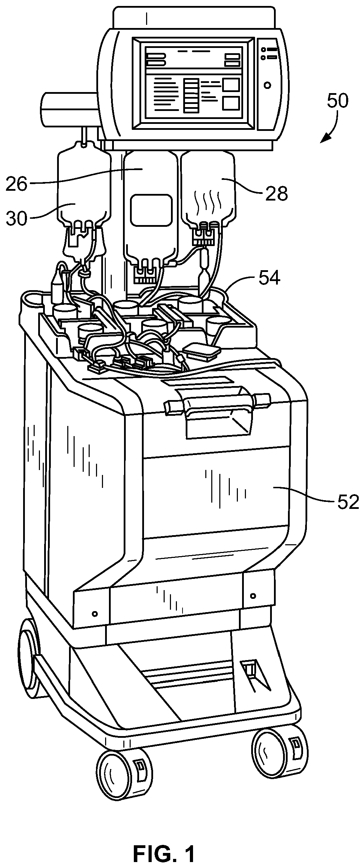

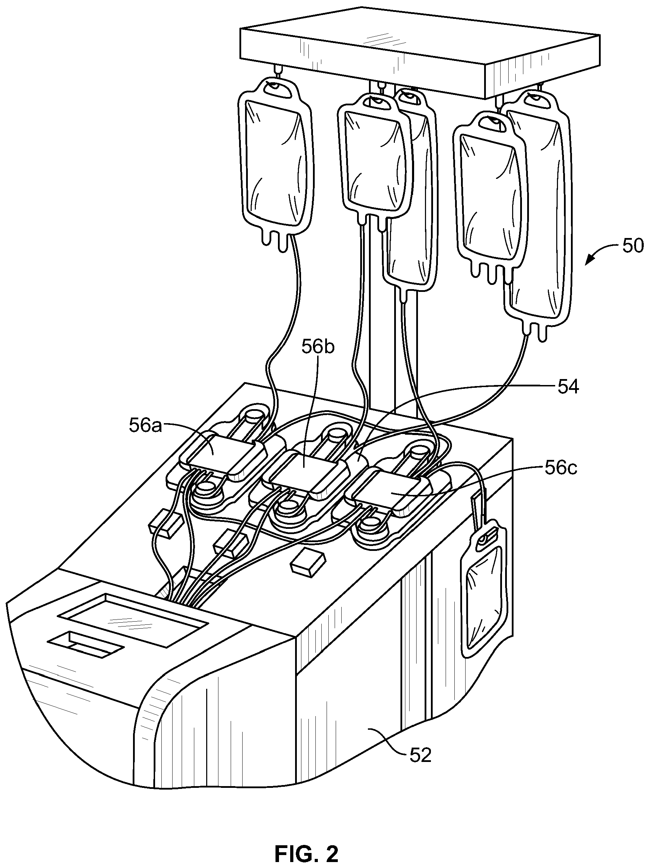

Figs. 1 and 2 show a representative separation device useful in the separation and collection of platelets. The separator 50 includes a hardware component 52 and a disposable processing kit 54 mounted thereon. In one embodiment, the separation principle used by the separator is based on centrifugation, but an automated separator based on a different separation principle may also be used.

With respect to the device shown in Figs. 1 and 2, a rotating centrifuge is housed within hardware component 52. Disposable fluid circuit 54 includes the plastic containers for holding fluid and tubing defining flow paths for movement of the blood, blood components and other fluids through the fluid circuit of 54. Disposable fluid circuit 54 includes one or more cassettes 56 (i.e., cassettes 56a, 56b and 56c shown in Figs. 2-3) which interface with the front panel of hardware component 52 and the peristaltic pumps located thereon. Cassettes 56a, 56b and 56c include inlet and outlet ports, internal flow paths and valve stations. A series of pneumatically or electrically operated valves under the control of a pre-programmed controller of hardware component 52 selectively allow and restrict flow through the flow paths of the cassette and ultimately through the tubing of fluid circuit 54. Cassettes 56a, 56b and 56c further include tubing loops that are engaged by the rollers of the peristaltic pumps thereby pumping fluid through fluid circuit 54. Further details of an automated separator suitable for use with the systems and methods described herein are set forth in U.S. Pat. Nos. 5,427,509; 6,312,607; 6,582,349 and U.S. Patent Application Publication 2009/0211987.

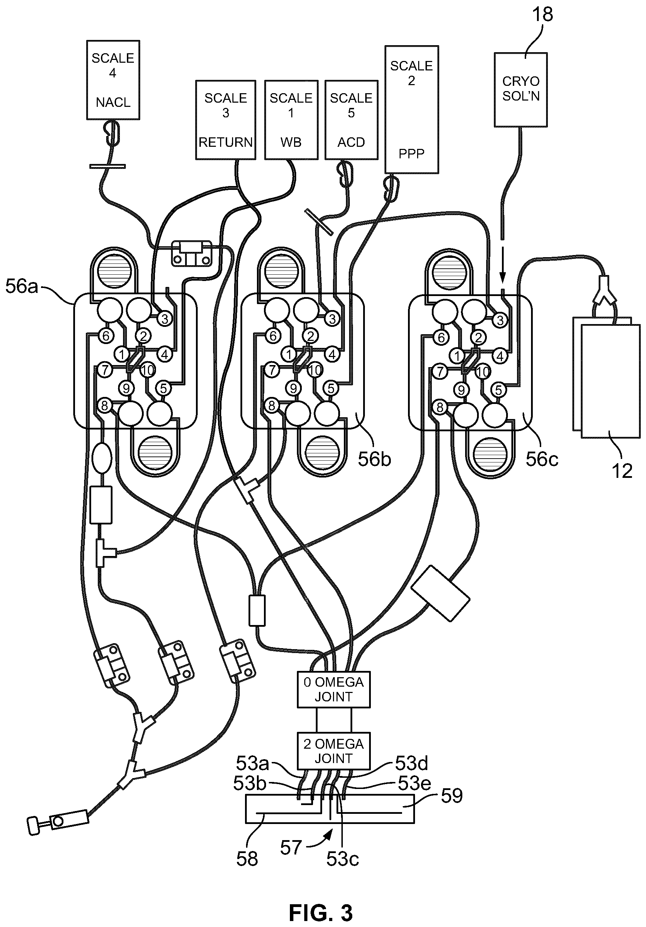

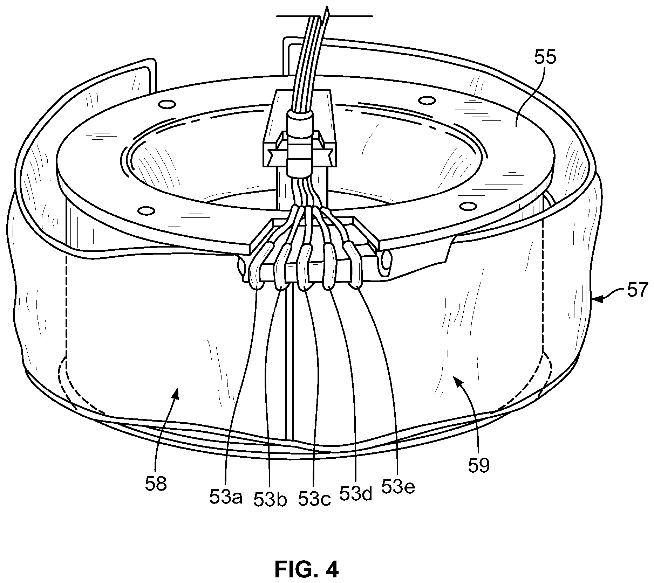

Disposable fluid circuit 54 further includes a processing chamber shown generally at 57 of Figs. 3-4 (which is mounted on a rotor/spool 55 of the centrifuge). Processing chamber 57 has a "first" sub-chamber 58 wherein blood or blood components are separated under the influence of centrifugal force (i.e., the "separation chamber) and a sub-chamber 59 where blood components from sub-chamber 58 can be further processed, separated and/or collected (i.e., the "concentration chamber"). Specifically, with regard to plateletpheresis, whole blood withdrawn from a donor is separated into platelet-rich plasma and red blood cells in sub-chamber 58 by centrifugation. While the red blood cells may be returned to the donor, the platelet rich plasma is expressed (during centrifugation) to sub-chamber 59 i.e., the concentration chamber where platelet-rich plasma is further separated into platelet-poor plasma and platelet concentrate. Residual (platelet-poor) plasma is removed from sub-chamber 59 and may be returned to the donor and/or collected in a container for later use. The platelets remaining in sub-chamber 59 are highly concentrated (or even "hyper-concentrated) and are suitable for cryopreservation. In one example, approximately 50 ml or less of concentrated platelets remain in sub-chamber 59. More preferably, approximately 30 ml of platelets at a concentration of about 3-50 x 10⁹/mL remain in sub-chamber 59. As shown in Fig. 4, processing chamber 57 includes multiple ports 53 a, b, c, d, e that are in fluid communication with sub-chambers 58 and 59 and allow for introduction and withdrawal of whole blood and components such as red blood cells, platelet-rich plasma, platelet poor plasma and, as will be described below, a cryopreservative solution.

In accordance with the present disclosure, rather than transfer the platelet concentrate from sub-chamber 59 to a separate collection container, platelet concentrate may be prepared for cryopreservation directly in sub-chamber 59. Thus, in accordance with the present disclosure, a container of cryopreservative solution 18 may be attached in a sterile manner at the completion of the platelet collection or preattached to fluid circuit 54 as shown in Fig. 3. For example, in one embodiment, cryopreservative solution may be attached to cassette 56c. Under the direction of the controller (which selectively effects the opening and closing of valves in cassette and rotation of pump rollers), pumps associated with cassette 56c may directly deliver a desired amount of cryopreservative solution to the platelet concentrate in sub-chamber 59. Alternatively, as will be described below, the cryopreservative solution may be combined with the platelets using a kit to which both sub-chamber 59 and a container of the cryopreservative solution are joined via a sterile docking method and device. In a still further alternative embodiment, as also described below, the cryopreservative solution may be added in the manner described above and as shown in Fig. 3, the platelet chamber separated from the fluid circuit and the cryopreserved product processed using the kit as described below.

The cryopreservative solution may be any solution that is compatible for use with platelets and can protect platelets during freezing. One well-known cryopreservative is dimethyl sulfoxide (DMSO). Where DMSO is the cryopreservative combined with platelets in sub-chamber 59, DMSO may comprise approximately 5-6% of the total fluid volume (platelet concentrate and cryopreservative).

In accordance with the present disclosure, once the platelet concentrate has been combined with the cryopreservative, sub-chamber 59 may be isolated (e.g. severed/disconnected) from the remainder of processing chamber 57 and/or fluid circuit 54. For example, with reference to Fig. 4, a liquid-tight seal may be formed between separation and concentration chambers 58 and 59 respectively. In addition, tubing ports 53 a-e that communicate with sub-chambers 58 and 59 may also be sealed. Sub-chamber 59 may then be severed from the remainder of processing chamber 57 and fluid circuit 54 along the formed seals described above.

Separated sub-chamber 59 may serve as the cryopreservation container. In that regard, at least sub-chamber 59 may be made of a material that is suitable for and can withstand the duration and cold storage temperatures commonly seen in the cryopreservation of platelets. In one embodiment at least sub-chamber 59 may be made of plasticized polyvinyl chloride. The plasticizer may be di-ethylhexyl terephthalate (DEHP) although other plasticizers may also be used with polyvinyl chloride. Other materials capable of withstanding cryopreservation may also include ethylene vinyl alcohol (EVA) or fluorinated ethylene propylene (FEP). It will be understood that while only the separated sub-chamber 59 will be stored under freezing conditions and the separated parts of the fluid circuit 54 and processing chamber 57 (such as the separation chamber 58) need not be made of material suitable for cryopreservation, such parts may be made of the same material as sub-chamber 59 for ease and efficiency of manufacture.

Platelets collected and prepared for cryopreservation in accordance with the above described non-therapeutic and non-surgical method are suitable for subsequent transfusion to a patient. A feasibility study was conducted to determine whether hyper-concentrated platelets obtained directly from the collection chamber of a plateletpheresis device can be used for the preparation of cryopreserved platelet products without the need for additional centrifugation and volume reduction.

### STUDY

**Study Design/Methods:** Single dose platelets (n = 10) were collected from healthy subjects using the Amicus^{®} Separator. Once collection was complete and the subject was disconnected from the device, the procedure was terminated prior to the product transfer step and the collection chamber was isolated. Hyper-concentrated platelets were resuspended in the residual plasma contained within the collection chamber (approximately 30 mL). Injection sites were sterile connected to the platelet collection chamber. A cryopreservation solution containing 10%DMSO (CryoStor^{®} CS10, BioLife Solutions^{®}) was then added to a final DMSO concentration of 6% (ave. total product volume: 62±1 mL). The collection chamber was then placed in a plastic overwrap bag and stored horizontally in a cardboard box within a -80°C freezer for a minimum of 2 weeks. For thawing, platelets were placed in a 37°C water bath for 5 minutes with gentle agitation. Temperature-equilibrated PAS-5 platelet additive solution (approx. 300 mL) was then slowly added with gentle mixing, and the platelets transferred to a 1L PL2410 platelet storage container. Platelets were stored at room temperature under constant agitation for up to 24 hours. Platelet quality was assessed just prior to freezing, immediately post thaw, and post PAS addition at times 0, 2, 6, and 24 hours.

In vitro parameter results are summarized in table below. Platelet yields ranged from (2.8 - 3.2) x1011. Discoid(%) for platelet morphology was >50% for up to 6 hrs post PAS addition. Platelets maintained HSR response through 24 hrs of storage, peaking at the 6 hr time point. Consistent with previous reports, platelets were activated immediately post thaw with no significant change observed over 24 hours. Thawed and reconstituted platelets were free of macroaggregrates. Microaggregrates, visible only by microscopy evaluation, tended to form between 2 - 6 hr time points, and were present throughout products by 24 hrs.

**TABLE 1**

| **Parameter, Mean ± SD (n = 10)** | **PreFreezing** | **PostThaw** | **Post PAS Addition (hr)** | | | |
|---|---|---|---|---|---|---|
| | | | **0** | **2** | **6** | **24** |
| Plt Conc (x 10⁹/L) | 4474 ± 431 | 4756 ± 189 | 801 ± 60 | 726 ± 52 | 659 ± 49 | 688 ± 51 |
| MPV (fL) | 7.9 ± 0.9 | 8.6 ± 0.8 | 8.9 ± 0.8 | 8.7 ± 0.7 | 8.1 ± 0.6 | 8.2 ± 0.6 |
| Morphology: Score (max 400) | 338 ± 9 | 286 ± 23 | 301 ± 12 | 295 ± 14 | 284 ± 6 | 242 ± 15 |
| Morphology: Discoid (%) | 67 ± 3 | 48 ± 10 | 56 ± 3 | 53 ± 4 | 51 ± 4 | 36 ± 5 |
| HSR (%) | NA | 27.5 ± 8.4* | 29.6 ± 2.1 | 38.6 ± 4.7 | 40.2 ± 2.2 | 23.8 ± 2.4 |
| CD62p (%) | 3.3 ± 5.0 | 46.4 ± 12.1 | 54.5 ± 17.4 | 52.6 ± 16.7 | 54.2 ± 19.1 | 51.8 ± 18.7 |

| | | | | | | |
|---|---|---|---|---|---|---|
| *n=7 | | | | | | |

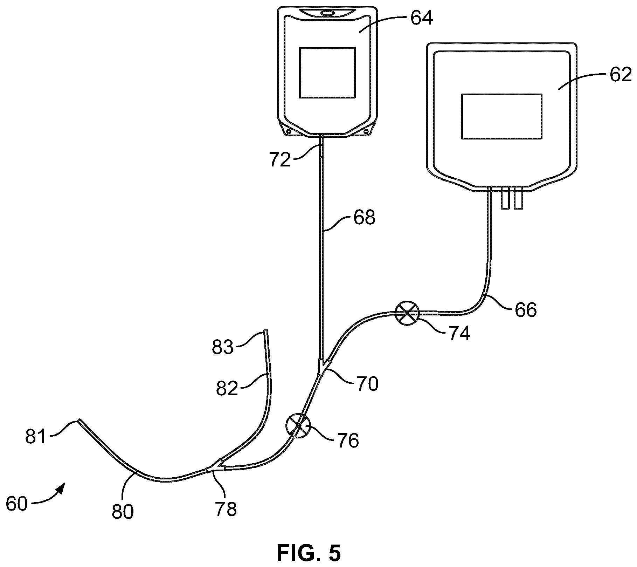

In accordance with the present disclosure, alternatively sub-chamber 59 may be joined to a pre-assembled cryopreservation kit 60 as shown in Fig. 5 and a container of cryopreservative solution. Kit 60 may include a platelet storage container 62 and a container 64 of platelet additive solution of the type described, for example, in WO 2012/139017. Each of containers 62 and 64 include tubing segment 66 and 68 which define flow paths for fluid communication between containers 62 and 64 and other components of kit 60. Tubing segments 66 and 68 are joined at branch connector 70 shown in Figure 5 and flow through tubing segments 66 and 68 may be regulated by conventional roller or Roberts-type clamps 74 and 76. In addition, flow path defined by tubing 68 may include a frangible connector 72 which when broken, establishes fluid communication between container 64 to the remainder of the kit. A second branch connector 78 is also shown in Figure 5. Branch connector 78 communicates with flow path 82 and flow path 80, each of which terminate in a sterile docking site for a container of cryopreservation solution (at docking site 83) and the sub chamber 59 (at docking site 81). In one embodiment, sterile docking may occur between docking site 81 and one of ports 53d or 53e that was sealed and severed from the remainder of the fluid circuit 54. Once sub-chamber 59 (with collected platelets therein) and the container of cryopreservative solution have been sterile docked to kit 60, the cryopreservative solution may be expressed into sub-chamber 59.

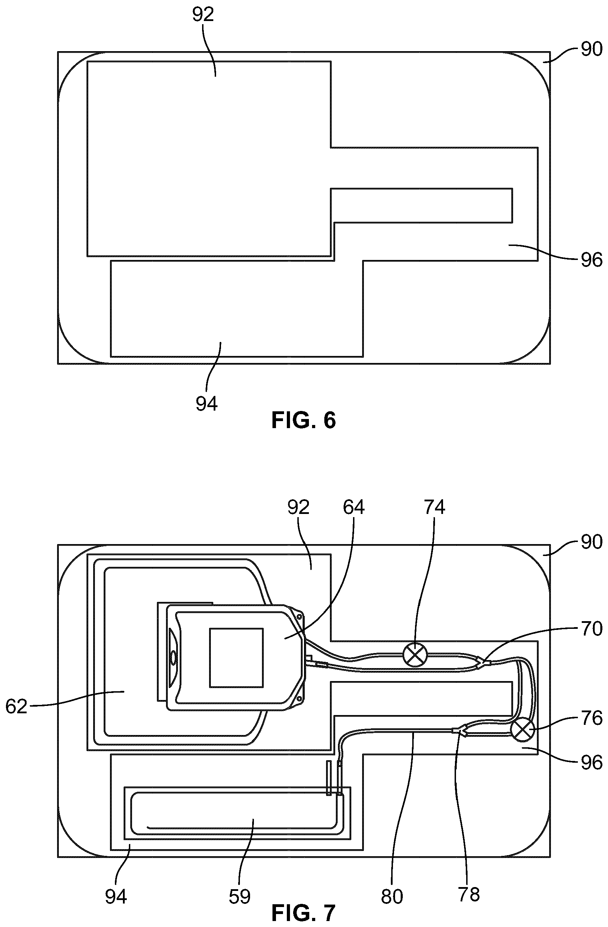

Kit 60 may be provided in a storage unit/cassette as shown for example in Figures 6-9. Storage unit 90 (shown in Figure 6), for example, not only serves as the packaging for kit 60 prior to use, but in accordance with the present disclosure, may also serve as the storage unit during freezing and thawing of the platelet product. Accordingly, storage unit 90 may be made of any material that is suitable for exposure to the freezing conditions of cryopreservation as well as thawing by submersion in a water bath or exposure to another thermal controlled environment. The material should be such that quick thawing of the platelet product (and frozen platelet additive solution) should not exceed five (5) minutes at approximately 37°c. Storage unit 90 may be made of either metal or a polymeric material or a combination of both. Examples of suitable materials include aluminum, stainless steel, polypropylene, polyethylene and polycarbonate.

As further shown in Figure 6, storage unit 90 may be generally rectangularly shaped and include inset areas/compartments 92, 94, and 96 to accommodate the containers and tubings of kit 60. For example, as shown in Figure 6, inset area 92 may be sized and shaped to contain platelet storage container 62 and platelet additive solution 64 (stacked on top). Inset area 94 may be sized and shaped to receive sub chamber 59 shown in Figure 6. Inset area 96 may be sized and shaped to accommodate the tubing segments, branch connectors as shown in Figure 6.

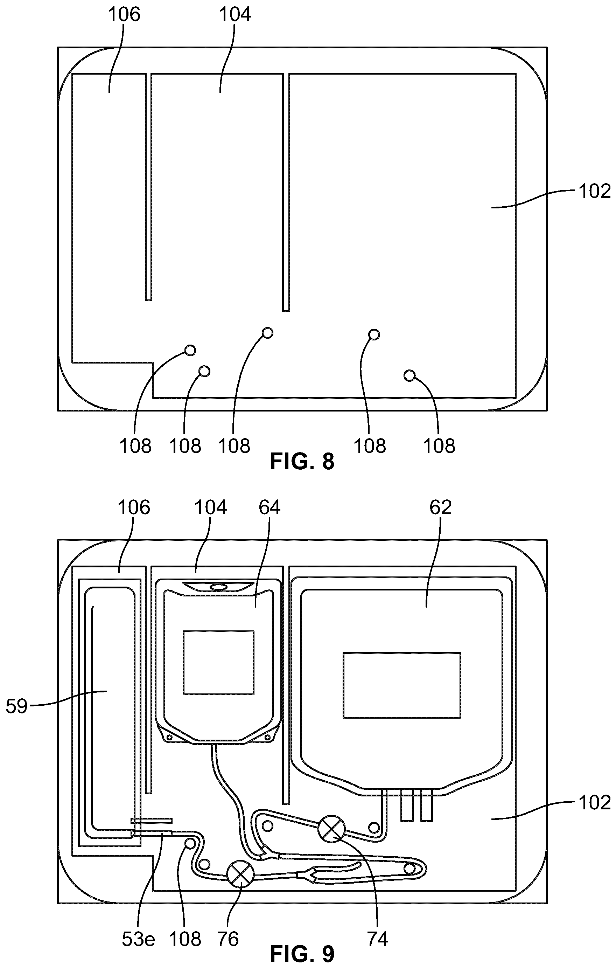

Figures 8 and 9 show an alternative embodiment of storage unit 100. Similar to storage unit 90 of Figures 6 and 7, storage unit 100 may be rectangularly shaped, made of material suitable for cryopreservation and thawing, and include inset areas/compartments 102, 104 and 106. In storage unit 100, inset areas 102 and 104 are arranged to allow for a side-by-side placement of platelet storage container 62 and additive solution container 64. Inset area 106 accommodates and allows for placement of chamber 59 after joinder of chamber 59 to the remainder to kit 60. Storage unit 100 may further include tubing guides 108 to thread and organize tubing segments 66, 68, 80, 82, and the associated components such as branch connectors and clamps.

In each example of the storage units 90 and 100, storage unit may include a base with the above described inset areas having an open top that may be closed with a lid. Organization of the containers and tubing segments and sub chamber 59 within storage units 90 and 100 are arranged to ensure adequate freezing of the platelet product in sub chamber 59 as well as complete thawing of the platelet product in sub chamber 59 and the platelet additive solution in container 64. Inasmuch as storage unit includes a flat bottom and flat lid, during cryopreservation, multiple storage units 90 or 100 may be stacked one on top of the other. In addition to the preassembled kit 60 with its pre-attached containers and docking sites, prior to cryopreservation, storage unit 90 or 100 may further include the container of the cryopreservative solution which, as described above, may be preattached to docking site 83 or provided as a standalone container for docking after platelet collection either directly to kit 60 or to the fluid circuit 54 as described above.

After the desired freezing period, storage units 90 and/or 100 may be removed from the freezer and thawed in a warm bath or other thermally controlled environment. Once the platelets in sub-chamber 59 and additive solution in container 64 have thawed, platelets may be expressed from sub-chamber 59 to platelet storage container 62. After breakage of frangible container 72, platelet additive solution or a portion thereof from container 64 may be expressed to platelet storage container 62. Some or all of the platelet additive solution may also be used to rinse sub-chamber 59 to ensure maximum platelet recovery. If only a portion of the additive solution is used to rinse sub-chamber 59, the remainder of the platelet additive solution from container 64 may then be added to platelet storage container 62.

In a further alternative, platelets may be collected and combined with the cryopreservative solution in the manner described above and shown in Fig. 3, while using kit 60 and/or storage unit 90 or 100 for the freezing and/or thawing and further processing of the platelets. In other words, a container of cryopreservative solution may be attached to the fluid circuit of Fig. 3 and, under the direction of the controller, pumps associated with the fluid circuit (e.g., cassette 56c) may directly deliver a desired amount of cryopreservative solution to the platelet concentrate in sub-chamber 59. Then, as previously described, sub-chamber 59 (now with platelets and the cryopreservative) may be isolated (e.g., severed/disconnected) from the remainder of processing chamber. Sub-chamber 59 may then be frozen first and then thawed prior to attachment to kit 60 for further processing with the additive solution and storage container 62.

Alternatively, sub-chamber 59 may be attached to kit 60 at, for example, connection site 81 and the entire kit frozen, as described above. Kit 60 would then be thawed and the platelets in sub-chamber 59 with the other (additive and storage) containers of kit 60, also as described above. In this embodiment, inasmuch as the cryopreservative solution is added by the automated plateletpheresis device of Fig. 3, there is no need to attach the cryopreservative solution at connection site 83.

It will be understood that the embodiments described above are illustrative of some of the applications and the principals of the present subject matter. Numerous modifications may be made by those of skilled in the art without departing from the scope of the claimed subject matter including those combinations of features that are individually disclosed or claimed herein. For these reasons the scope hereof is not limited to the above description.

## Claims

1. A cryopreservation kit (60) comprising:
a. a fluid circuit including a container of a platelet additive solution (64) and a platelet storage container (62) and tubing (66, 68) interconnecting said platelet storage container (62) and said additive solution container (64) and establishing a flow paths therebetween; and
b. a storage unit (90) comprising a housing including insets/compartments (92, 94, 96) for holding a container of platelets (59), the container of said platelet additive solution (64) and said platelet storage container (62), said housing comprising a waterproof material that allows frozen platelets to thaw.

2. The cryopreservation kit of Claim 1 comprising a compartment (94) for holding said platelet container (59) and a compartment (92) for holding at least one of said additive solution container (64) and said platelet storage container (62).

3. The cryopreservation kit of any one of Claims 1 and 2 wherein said fluid circuit includes at least one sterile docking site (81, 83), in particular a sterile docking site (83) for a container of a cryopreservative solution.

4. The cryopreservation kit of Claim 3 wherein said kit includes a sterile docking site (81) for a container of platelet concentrate.

5. The cryopreservation kit of any one of Claims 1 through 4 wherein said housing includes an open top and a lid.

6. The cryopreservation kit of any one of Claims 1 through 5 wherein said housing comprises tubing guides.

7. The cryopreservation kit of Claim 6 wherein said tubing guides comprise compartments for accommodating said tubing.

8. The cryopreservation kit of any one of Claims 6 through 7 wherein said guides comprises a plurality of upstanding members around which said tubing is threaded.

9. The cryopreservation kit of any one of Claims 1 through 8 wherein said housing is made of a material that allows a cryopreserved platelet product to thaw in approximately 5 minutes or less at a temperature of approximately 37°C.

10. The cryopreservation kit of any one of Claims 1 through 9 wherein said housing is made of a polymeric material that is suitable for exposure to temperatures up to about -80°C without damage.

11. A non-therapeutic and non-surgical method for preparing cryopreserved blood product comprising
a) introducing whole blood into a centrifugal chamber (57), and said chamber (57) comprising a first sub-chamber (58) and a second sub-chamber (59);
b) concentrating platelets in said second sub-chamber (59) );
c) introducing a cryopreservative solution into said second sub-chamber (59);
d) isolating said second sub-chamber (59) from said first sub-chamber (58);
e) attaching said isolated second sub-chamber to the kit of any one of Claims 1-10; and
f) freezing said second sub-chamber (59) for a selected period of time.

12. The method of Claim 11 wherein said centrifugal chamber (57) is part of an automated fluid processing device, said device comprising a controller configured to effect the processing of a blood product and for effecting the introduction of said cryopreservative solution to said concentrated platelets.

13. The method of any one of Claims 11 and 12 further comprising attaching said isolated second sub-chamber (59) to a kit (60) comprising an additive solution container (64) and a platelet storage container (62), in particular prior to freezing.

14. The method of any one of Claims 11 through 13 further comprising thawing said platelets.

15. The method of any one of Claims 11 through 13 comprising freezing said platelets after attaching said second sub-chamber to said kit.

## Patentansprüche

1. Kryokonservierungs-Kit (60), umfassend:
a. einen Fluidkreislauf, der einen Behälter mit einer Thrombozyten-Additivlösung (64) und einen Thrombozyten-Lagerungsbehälter (62) sowie Schläuche (66, 68) umfasst, die den genannten Thrombozyten-Lagerungsbehälter (62) und den genannten Behälter mit Additivlösung (64) miteinander verbinden und dazwischen Strömungswege herstellen; und
b. eine Lagereinheit (90) umfassend ein Gehäuse mit Einsätzen/Fächern (92, 94, 96) zum Aufnehmen eines Thrombozytenbehälters (59), des Behälters mit der genannten Thrombozyten-Additivlösung (64) und des genannten Thrombozyten-Lagerungsbehälters (62), wobei das Gehäuse ein wasserdichtes Material umfasst, das das Auftauen gefrorener Thrombozyten ermöglicht.

2. Kryokonservierungs-Kit nach Anspruch 1, umfassend ein Fach (94) zum Aufnehmen des genannten Thrombozytenbehälters (59) und ein Fach (92) zum Aufnehmen mindestens eines von dem genannten Behälter mit Additivlösung (64) und dem genannten Thrombozyten-Lagerungsbehälter (62).

3. Kryokonservierungs-Kit nach einem der Ansprüche 1 und 2, wobei der genannte Fluidkreislauf mindestens eine sterile Andockstelle (81, 83) umfasst, insbesondere eine sterile Andockstelle (83) für einen Behälter mit einer Kryokonservierungslösung.

4. Kryokonservierungs-Kit nach Anspruch 3, wobei das genannte Kit eine sterile Andockstelle (81) für einen Behälter mit Thrombozytenkonzentrat umfasst.

5. Kryokonservierungs-Kit nach einem der Ansprüche 1 bis 4, wobei das genannte Gehäuse eine offene Oberseite und einen Deckel umfasst.

6. Kryokonservierungs-Kit nach einem der Ansprüche 1 bis 5, wobei das genannte Gehäuse Schlauchführungen umfasst.

7. Kryokonservierungs-Kit nach Anspruch 6, wobei die genannten Schlauchführungen Fächer zum Aufnehmen der genannten Schläuche umfassen.

8. Kryokonservierungs-Kit nach einem der Ansprüche 6 bis 7, wobei die genannten Führungen eine Vielzahl aufstehender Elemente umfassen, um welche die genannten Schläuche geführt sind.

9. Kryokonservierungs-Kit nach einem der Ansprüche 1 bis 8, wobei das genannte Gehäuse aus einem Material hergestellt ist, das es einem kryokonservierten Thrombozytenprodukt ermöglicht, bei einer Temperatur von ungefähr 37 °C in ungefähr 5 Minuten oder weniger aufzutauen.

10. Kryokonservierungs-Kit nach einem der Ansprüche 1 bis 9, wobei das genannte Gehäuse aus einem polymeren Material hergestellt ist, das geeignet ist, Temperaturen bis zu etwa -80 °C ohne Beschädigung ausgesetzt zu werden.

11. Nicht-therapeutisches und nicht-chirurgisches Verfahren zur Herstellung eines kryokonservierten Blutprodukts, umfassend
a) Einführen von Vollblut in eine Zentrifugalkammer (57), wobei die genannte Kammer (57) eine erste Unterkammer (58) und eine zweite Unterkammer (59) umfasst;
b) Konzentrieren von Thrombozyten in der genannten zweiten Unterkammer (59);
c) Einführen einer Kryokonservierungslösung in die genannte zweite Unterkammer (59);
d) Isolieren der genannten zweiten Unterkammer (59) von der genannten ersten Unterkammer (58);
e) Anbringen der genannten isolierten zweiten Unterkammer an das Kit nach einem der Ansprüche 1-10; und
f) Einfrieren der genannten zweiten Unterkammer (59) für einen ausgewählten Zeitraum.

12. Verfahren nach Anspruch 11, wobei die genannte Zentrifugalkammer (57) Teil einer automatisierten Fluidverarbeitungsvorrichtung ist, wobei die genannte Vorrichtung einen Controller umfasst, der dazu konfiguriert ist, die Verarbeitung eines Blutprodukts zu bewirken und das Einführen der genannten Kryokonservierungslösung in die genannten konzentrierten Thrombozyten zu bewirken.

13. Verfahren nach einem der Ansprüche 11 und 12, weiter umfassend das Anbringen der genannten isolierten zweiten Unterkammer (59) an ein Kit (60), das einen Behälter mit Additivlösung (64) und einen Thrombozyten-Lagerungsbehälter (62) umfasst, insbesondere vor dem Einfrieren.

14. Verfahren nach einem der Ansprüche 11 bis 13, weiter umfassend das Auftauen der genannten Thrombozyten.

15. Verfahren nach einem der Ansprüche 11 bis 13, umfassend das Einfrieren der genannten Thrombozyten, nachdem die genannte zweite Unterkammer an das genannte Kit angebracht wurde.

## Revendications

1. Kit de cryoconservation (60) comprenant :
a. un circuit fluidique comprenant un récipient d'une solution additive plaquettaire (64) et un récipient de stockage de plaquettes (62), ainsi que des tubulures (66, 68) interconnectant ledit récipient de stockage de plaquettes (62) et ledit récipient de solution additive (64) et établissant des chemins d'écoulement entre eux ; et
b. une unité de stockage (90) comprenant un boîtier comportant des inserts/compartiments (92, 94, 96) destinés à contenir un récipient de plaquettes (59), le récipient de ladite solution additive plaquettaire (64) et ledit récipient de stockage de plaquettes (62), ledit boîtier comprenant un matériau étanche à l'eau qui permet à des plaquettes congelées de décongeler.

2. Kit de cryoconservation selon la revendication 1, comprenant un compartiment (94) destiné à contenir ledit récipient de plaquettes (59) et un compartiment (92) destiné à contenir au moins l'un dudit récipient de solution additive (64) et dudit récipient de stockage de plaquettes (62).

3. Kit de cryoconservation selon l'une quelconque des revendications 1 et 2, dans lequel ledit circuit fluidique comprend au moins un site de connexion stérile (81, 83), en particulier un site de connexion stérile (83) pour un récipient d'une solution cryoconservatrice.

4. Kit de cryoconservation selon la revendication 3, dans lequel ledit kit comprend un site de connexion stérile (81) pour un récipient de concentré plaquettaire.

5. Kit de cryoconservation selon l'une quelconque des revendications 1 à 4, dans lequel ledit boîtier comprend un dessus ouvert et un couvercle.

6. Kit de cryoconservation selon l'une quelconque des revendications 1 à 5, dans lequel ledit boîtier comprend des guides de tubulure.

7. Kit de cryoconservation selon la revendication 6, dans lequel lesdits guides de tubulure comprennent des compartiments destinés à recevoir lesdites tubulures.

8. Kit de cryoconservation selon l'une quelconque des revendications 6 à 7, dans lequel lesdits guides comprennent une pluralité d'éléments saillants autour desquels lesdites tubulures sont enfilées.

9. Kit de cryoconservation selon l'une quelconque des revendications 1 à 8, dans lequel ledit boîtier est fabriqué dans un matériau qui permet à un produit plaquettaire cryoconservé de décongeler en environ 5 minutes ou moins à une température d'environ 37 °C.

10. Kit de cryoconservation selon l'une quelconque des revendications 1 à 9, dans lequel ledit boîtier est fabriqué dans un matériau polymérique approprié pour être exposé à des températures allant jusqu'à environ -80 °C sans dommage.

11. Procédé non thérapeutique et non chirurgical de préparation d'un produit sanguin cryoconservé, comprenant :
a) l'introduction de sang total dans une chambre centrifuge (57), ladite chambre (57) comprenant une première sous-chambre (58) et une seconde sous-chambre (59) ;
b) la concentration de plaquettes dans ladite seconde sous-chambre (59) ;
c) l'introduction d'une solution cryoconservatrice dans ladite seconde sous-chambre (59) ;
d) l'isolement de ladite seconde sous-chambre (59) par rapport à ladite première sous-chambre (58) ;
e) la fixation de ladite seconde sous-chambre isolée au kit selon l'une quelconque des revendications 1 à 10 ; et
f) la congélation de ladite seconde sous-chambre (59) pendant une période de temps sélectionnée.

12. Procédé selon la revendication 11, dans lequel ladite chambre centrifuge (57) fait partie d'un dispositif automatisé de traitement de fluide, ledit dispositif comprenant un contrôleur configuré pour effectuer le traitement d'un produit sanguin et pour effectuer l'introduction de ladite solution cryoconservatrice dans lesdites plaquettes concentrées.

13. Procédé selon l'une quelconque des revendications 11 et 12, comprenant en outre la fixation de ladite seconde sous-chambre isolée (59) à un kit (60) comprenant un récipient de solution additive (64) et un récipient de stockage de plaquettes (62), en particulier avant la congélation.

14. Procédé selon l'une quelconque des revendications 11 à 13, comprenant en outre la décongélation desdites plaquettes.

15. Procédé selon l'une quelconque des revendications 11 à 13, comprenant la congélation desdites plaquettes après la fixation de ladite seconde sous-chambre audit kit.
